# EUROPÄISCHE PATENTSCHRIFT

(11) **EP 0 628 385 B1**
(45) Veröffentlichungstag und Bekanntmachung des Hinweises auf die Patenterteilung: **19.04.1995**
(21) Anmeldenummer: 93109348.8
(22) Anmeldetag: 11.06.1993
(51) Int. Cl.: B25J 3/04, B25J 15/00, B25J 9/10

(54) **Elastischer Körper**
Elastic body
Corps élastique

(43) Veröffentlichungstag der Anmeldung: 14.12.1994
(73) Patentinhaber: Daum, Wolfgang, D-19061 Schwerin (DE)
(72) Erfinder: Daum, Wolfgang, D-19061 Schwerin (DE)

(56) Entgegenhaltungen:
- GB-A- 2 013 617
- US-A- 4 792 173
- 'PROCEEDINGS 1990 IEEE INTERNATIONAL CONFERENCE ON ROBOTICS AND AUTOMATION' Mai1990 , IEEE COMPUTER SOCIETY FUKUDA ET AL. :DISTRIBUTED TYPE OF ACTUATORS BY SMA AND ITS APPLICATION TO UNDERWATER MOBILE ROBOTIC MECHANISM PAGES 1316 TO 1321 :

## Beschreibung

Die Erfindung betrifft eine Vorrichtung zum Greifen nach dem Oberbegriff des Anspruchs 1, eine Vorrichtung zum Abtasten der Formänderung eines Körpers nach dem Oberbegriff des Anspruchs 5, sowie ein Verfahren zur Verwendung dieser Vorrichtung gemäß Oberbegriff von Anspruch 7. Solche Vorrichtungen b.z.w. ein solches Verfahren sind z.B. in der GB-A-2 013 617 beschrieben.

### Problem

1. Bewegungsabläufe technischer Vorrichtungen werden heutzutage durch einzelne oder mehrere Motoren oder Aktoren diverser Art betrieben. Bewegungsveränderungen werden dann durch Hinzufügen von z.B. Getrieben, Hebeln oder Flaschenzügen erreicht, deren Bewegungsabläufe während der Konstruktionen festgelegt wurden und nachträglich nur durch Umbau verändert werden können. Der Bewegungsablauf ist durch die Konstruktion festgelegt.
   Im Unterschied zur vom Menschen geschaffenen Technik werden in der Natur Bewegungen durch z.B. Muskeln erzeugt. Diese bestehen aus einer Vielzahl von Zellen, die sich zusammenziehen können. Dabei sind in einem arbeitenden Muskel selten alle Muskelzellen gleichzeitig tätig, sondern immer nur jene und so viele, wie zu der Bewegungsausführung benötigt werden. Dadurch ist ein Muskel viel flexibler und kann auch unvorhergesehene Bewegungen ausführen. Denn die Bewegungsabläufe werden nicht vorher konstruktiv festgelegt, sondern erlernt. Der Bewegungsmechanismus eines Muskels ist redundant. D.h., fallen Muskelzellen aus, finden sich immer andere, die anstelle dieser kontrahieren, so daß die erzielte Gesamtbewegung des Muskels wieder stimmt.
   Würde man muskelähnliche mechanische Elemente besitzen, so könnte man eine ganze Reihe neuer Vorrichtungen, wie z.B. Nachbauten menschlicher Gliedmaßen, fertigen. Mechanische Elemente, die in ihrer Bewegung nicht durch die Konstruktion sondern nur durch ein sie steuerndes Comuterprogramm festgelegt sind, würden viele technische Probleme lösen. Zumal dann, wenn sich die Aufgabenstellung des mechanischen Elementes bzw. die der Vorrichtung, zu der das Element gehört, ändert.
   Die Forderung nach der Redundanz technischer Systeme wird heutzutage durch den mehrfachen Einbau, in kleiner Zahl, gleicher Bauelemente in diese Vorrichtung erreicht. Die Elemente selbst sind dabei nicht redundant. Es wäre daher wünschenswert, mechanische Elemente zu haben, die selbstredundant sind.
   In der Informatik werden heute Algorithmen erarbeitet, die selbstlernend sind. Die Umsetzung solcher Algorithmen in die mechanische Welt macht vor allem dann Sinn, wenn die Bewegungsmöglichkeiten der Vorrichtungen nicht durch die Konstruktion starr festgelegt, sondern flexibel sind.
2. Bewegungsabläufe technischer Vorrichtungen werden heutzutage mittels einzelner oder kleinerer Mengen von Sensoren bestimmt. Die Sensoren befinden sich dabei an den für einen speziellen Regelablauf einer speziellen Konstruktion wichtigen Stellen. Da die Bewegungsabläufe durch die Konstruktionen festgelegt wurden, reicht die geringe Anzahl festpositionierter Sensoren für die Erfassung der Bewegungen aus.
   Dies wäre jedoch nicht der Fall, hätte man mechanische Elemente, deren Bewegungen nicht konstruktiv festgelegt, sondern flexibel und durch einen Computeralgorithmus bestimmt wären. Die Erfassung der momentanen Stellung und Bewegung solcher Elemente mit herkömmlichen Mitteln würde sich als schwierig bis unmöglich gestalten und könnte den Charakter der Elemente sogar verfälschen. Für ein solches Element wäre es daher wünschenswert, ein Sensorsystem zu haben, welches alle Form- und Bewegungsänderungen aufnehmen kann.
3. Der Mensch hat sich seine technische Umwelt, in der er lebt, so geschaffen, daß er mit seinen Gliedern in dieser optimal aggieren kann. Roboter, die ihm Aufgaben in dieser Umwelt abnehmen, werden besonders dann sinnvoll arbeiten, wenn sie menschenähnlicher Gestalt sind und sich ebenso bewegen. Daher müssen besonders die Roboterhände den Menschenhänden entsprechen.

### Aufgabe und Ziel

Aufgabe und Ziel der Erfindung ist es, einen elastischen Körper zu schaffen, der seine Form nahezu kontinuierlich ändern kann, durch Computeralgorithmen gesteuert wird und als mechanisches Element z.B. zur Kraft- und Bewegungserzeugung dient. Dabei liegt das Augenmerk auch auf der Schaffung einer Art organischen Körpers, in der Hinsicht, daß dieser durch selbstlernende Algorithmen steuerbar ist. Weiterhin ist es das Ziel, die Formänderung eines solchen sich selbst verformenden Elementes zu messen.

Es ist ein weiteres Ziel dieser Erfindung, Roboterhände mit einer Master-Slave-Steuerung zu erstellen, die ihre Antriebe in sich selbst tragen und aufgrund ihrer elastischen Bauart vom Menschen als angenehm empfunden werden.

Technische Vorrichtungen, die durch eine Vielzahl von Aktuatoren betrieben werden, sind zwar bekannt, wie z.B. in DE-A-29 15 313 dargelegt. Deren Aktuatoren sind jedoch nicht in ein elastisches Material eingelagert, so daß eine Vielfalt von nicht vorherbestimmten Bewegungen sondern nur konstruktiv festgelegte ausgeführt werden können.

Technische Vorrichtungen, die elastische Körper als wesentliche Bestandteile aufweisen, sind zwar bekannt, wie z.B. in DE-A-40 33 089 dargelegt. Diese werden jedoch nicht durch eine Vielzahl von einzeln oder in Gruppen ansteuerbaren Aktuatoren betrieben. Auch sie zeichnen sich nicht durch eine unvorherbestimmte Bewegungsvielfalt aus.

Handnachbauten, die sich ferngesteuert lenken lassen, sind zwar bekannt, wie z.B. in WO-A-90/04370 dargelegt. Deren Anzahl von Stellelementen ist jedoch so begrenzt, daß dies nur zum einen konstruktiv vorbestimmte und zum anderen sehr abrupte Bewegungen ausführen können. Die momentane Stellung der Gliedmaßen wird nicht gemessen.

### Lösung

Die Ziele dieser Erfindung werden durch die Patentansprüche 1, 5 und 7 gelöst. Diese sollen anhand folgender Figuren erläutert werden :
- Fig. 1: a - plattenförmiger elastischer Körper mit aus Formgedächtnismaterial gefertigten Spiralen als Aktuatoren im Grundzustand,
b - im Schaltzustand;
- Fig. 2: a - Anordung von Formgedächtnisdrähten zu einer zweidimensionalen Matrix,
b - Formgedächtnisdraht mit Halterung;
- Fig. 3: a - Kombination zweier Matrizen mit unterschiedlicher Wirkrichtung der Formgedächtnisdrähte,
b - Querschnitt durch fünf Matrizen bei einer Biegung,
c - Bewegungsablauf eines plattenförmigen, elastischen Körpers;
- Fig. 4: Anwendungsbeispiel : selbstdichtender Deckel, a - offen, b - geschlossen, c - dichtend;
- Fig. 5: Anwendungsbeispiel : künstliche Hand;
- Fig. 6: a - Aktuator aus einem Bimetall,
b - elektromagnetisch arbeitender Aktuator mit innerer Bewegung,
c - elektromagnetisch arbeitende Aktuatoren die sich gegenseitig abstoßen bzw. anziehen;
- Fig. 7: Querschnitt durch Schalter in einem elastischen Körper, die schalten, wenn der Körper a - gestaucht oder b - gebogen wird;
- Fig. 8: Querschnitt durch Schalter in einem elastischen Körper, die schalten, wenn der Körper gebogen wird;
- Fig. 9: Querschnitt durch einen Fingerteil eines Datenhandschuhs, der ein elastischer Körper mit einer Vielzahl von Schaltern ist, die gleich derer aus Fig. 8 aufgebaut sind und
- Fig. 10: Flächendrucksensor in a - Explosionsdarstellung und b - Querschnitt eines Schalterelementes.

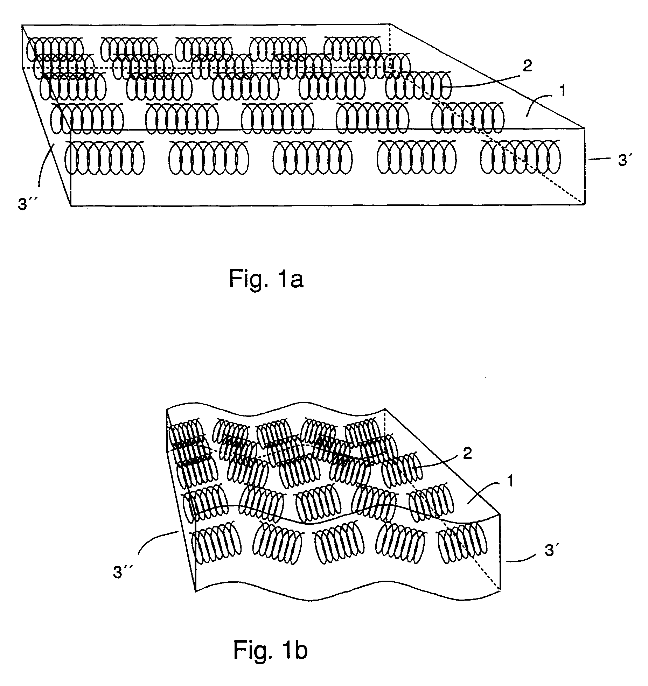

In Fig. 1 ist ein z.B. aus Silikonkautschuk gefertigter elastischer Körper (1) dargestellt. In diesen sind Spiralfedern (2) eingegossen, die sich nach einem weiter unten erläuterten Mechanismus zusammenziehen können. Sie werden hier Aktuatoren genannt, da sie im Schaltzustand eine Bewegung bzw. Aktion ausführen. In Fig. 1a liegen diese im ungeschalteten Grundzustand vor. Geschaltet, Fig. 1b, ziehen sie sich und damit den Körper zusammen, die Form des Körpers (1) ändert sich, er wellt und die Grenzflächen (3) und (4) nähern sich.

Formgedächtnismetalle, wie z.B. bestimmte Titan-Nickel-Legierungen, ändern bei bestimmten Temperaturen durch eine Phasenumwandlung ihre äußere Form. Die in Fig. 1 gezeigten Spiralen sind aus solch einem Material gefertigt. Der Schaltzustand wird durch Temperaturänderung erreicht.

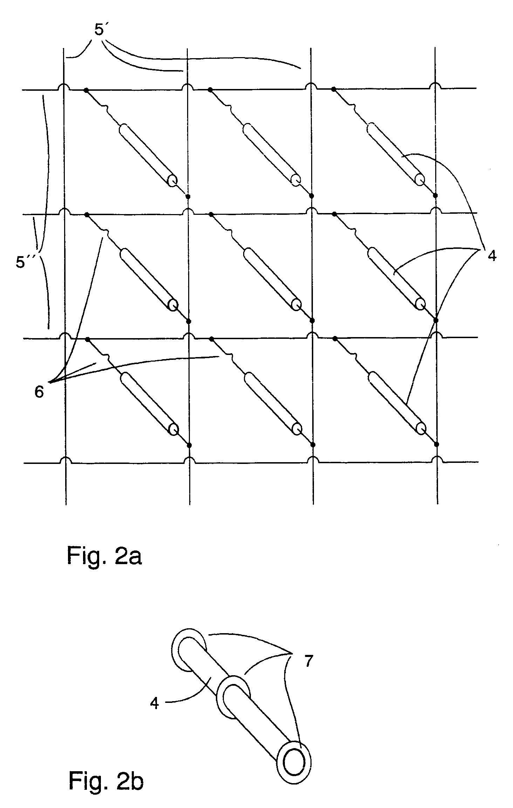

Fig. 2a stellt einen Teil einer zweidimensionalen Kraftelementmatrix aus Formgedächtnisdrähten dar. Die Drähte lassen sich hier durch Wahl der richtigen Schaltleitungen (5') und (5'') einzeln ansteuern und direkt aufheizen. Dabei ist der Widerstand der Schaltleitungen (5') und (5'') so bemessen, daß sich nur die Formgedächtnisdrähte aufheizen. Denkbar wäre jedoch auch eine indirekte Beheizung. Durch Entspannvorrichtungen (6) wird die Matrix flexibel für Bewegungen. Mit den z.B. ringförmig ausgeführten Halterungen (7), Fig. 2b, können die Formgedächtnisdrähte (4) bei der Formveränderung in dem elastischen Material des Körpers festhalten werden.

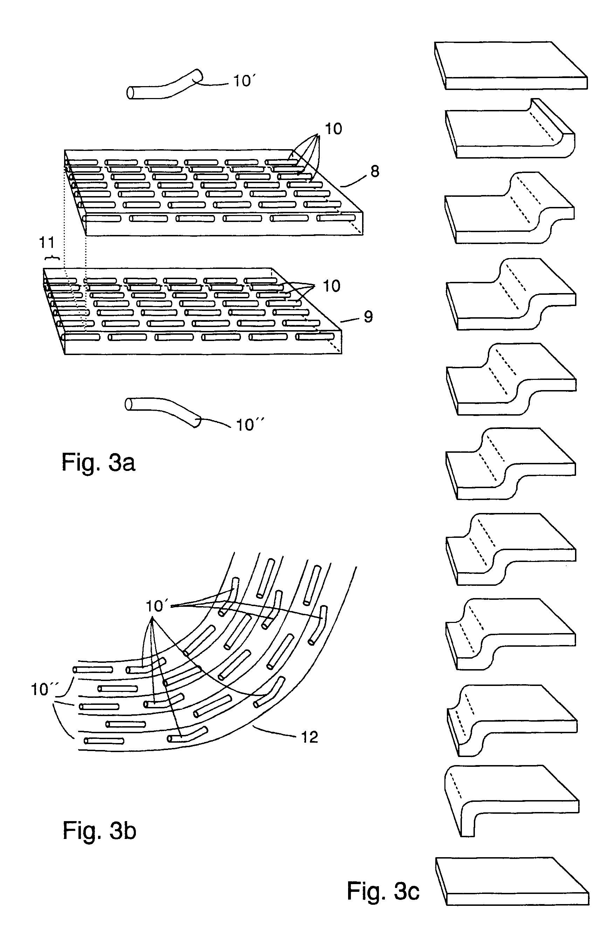

Anhand der Fig. 3 soll gezeigt werden, wie mit einer Einzelaktuatoransteuerung eine nahezu stetige Bewegung ausgeführt werden kann. Dabei werden ebenso wie in allen anderen Figuren die Schaltleitungen und die Halterungen nicht gezeigt.

Mehrere der in Fig. 2a dargestellten zweidimensionalen Matrizen sind in einen gummielastischen Körper vergossen. Die durch die Matrizen gebildeten Ebenen sind um je ca. eine halbe Drahtlänge (11) gegeneinander versetzt . Fig. 3a verdeutlicht dies an zwei Lagen (8) und (9). Die Ebenen brauchen dabei keinen realen, sondern nur gedanklich ordnenden Charakter zu haben. Stellvertretend für alle Elemente einer Ebene ist anhand der Biegung der Drähte (10') und (10'') die Wirkungsrichtung der Aktuatoren der Ebenen (8) und (9) gezeigt. Fig. 3b zeigt einen Schnitt durch einen Teil eines solchen Mehrlagensystems. Dabei wurden die Aktuatoren (10) geschaltet, so daß sie sich und damit den Körper (12) biegen. Wären andere Aktuatoren geschaltet, ergäben sich andere Formveränderungen des Körpers (12). Daher kann durch fortlaufendes Schalten einzelner oder Gruppen der Aktuatoren eine fortlaufende Bewegung, wie sie z.B. in Fig. 3c in 11 aufeinanderfolgenden Zeitintervallen dargestellt ist, erreicht werden. Da solche Formgedächtnismetallteile ihre Form sehr abrupt ändern, ist die Bewegung desto kontinuierlicher, je höher die Anzahl der Aktuatoren ist. Die gezeigten Matrixfelder könnten auch dreidimensional ineinanderliegen. Hieraus ergeben sich vielfältigste Bewegungsabläufe. Sie beruhen alle auf dem hier angemeldeten Bewegungsprinzip. Die hohe Anzahl von eingelagerten Aktuatoren verleiht dem elastischen Körper Redundanz. Denn man findet beim Ausfall von Aktuatoren immer andere, die anstelle dieser geschaltet werden können, so daß die Gesamtbewegung des elastischen Körpers unverändert bleibt.

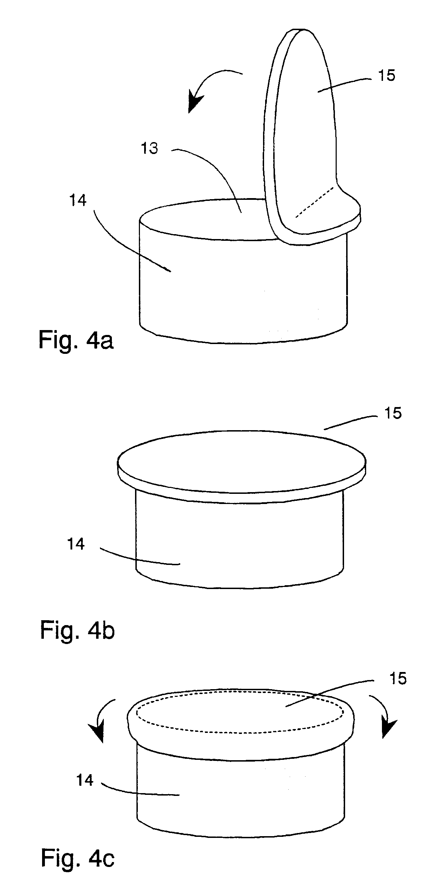

In Fig. 4 ist ein Anwendungsbeispiel gezeigt. Auf einer Öffnung (13) eines Gefäßes (14) befindet sich ein Deckel (15), der als ein elastischer Körper mit einer Vielzahl von eingelagerten Aktuatoren ausgeführt ist. In Fig. 4a ist dieser Deckel geöffnet. Durch den oben beschriebenen Bewegungsmechanismus legt sich dieser selbständig auf die Öffnung, Fig. 4b, und verschließt diese dichtend, Fig. 4c.

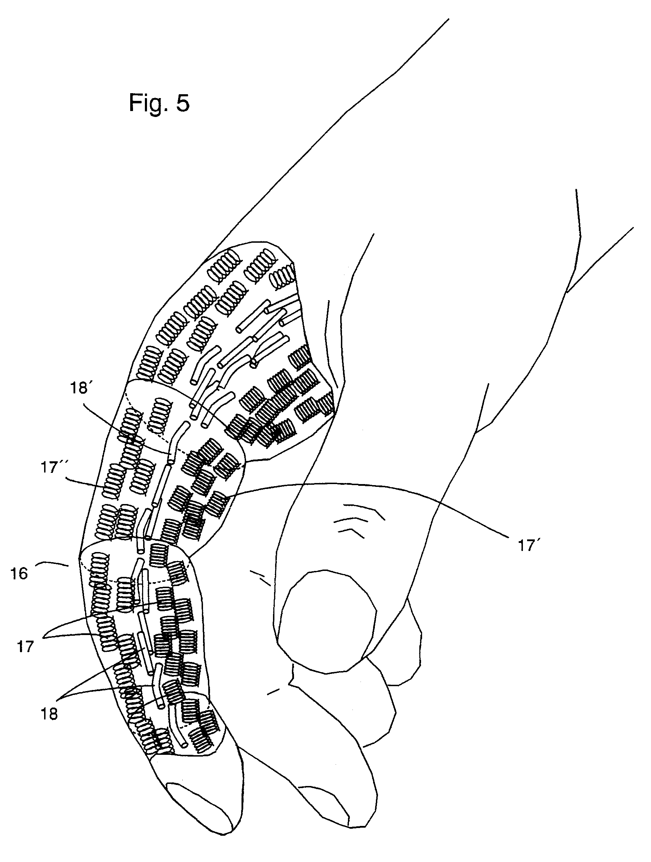

Eine weitere Anwendung so gearteter elastischer Körper sind Nachbildungen von Muskeln. Hierzu zeigt Fig. 5 eine aus z.B. Silikonkautschuk gefertigte künstliche Hand. An dem in Durchsicht dargestellten Zeigefinger (16) ist zu erkennen, daß die Aktuatoren (17) ohne strenge Ordnung in das Material eingelassen sind. Diese Aktuatoren sind hier aus Formgedächtnismaterial gefertigte Spiralen (17). Gezeigt ist ein gebeugter Zustand des Zeigefingers, der durch das Zusammenziehen der Spiralen (17') der Fingerunterseite oder auch Strecken der Spiralen (17'') der Fingeroberseite hervorgerufen ist. Eventuelle Stützdrähte sind nicht gezeigt. Hierbei wäre auch eine Bauweise denkbar, die sich aus einzelnen Muskelsträngen zusammensetzt. Die weiter unten beschriebenen Schalt- oder Sensorelemente (18) geben dem steuernden Rechner die Stellung des Fingers (16) an.

Die genaue Lage der Aktuatoren braucht nicht wie im Beispiel der Fig. 3 bekannt zu sein. Durch Probieren und Beobachten mit z.B. graphischer Datenauswertung kann der steuernde Rechner die Bewegungen erlernen. Dieser Lernprozeß ist deshalb möglich, da der elastische Körper mit seiner Vielzahl von Aktuatoren dem Steueralgorithmus die Möglichkeit gibt, sich genau die Aktuatoren auszuwählen, die zur angestrebten Bewegung benötigt werden. Je höher die Anzahl der Aktuatoren ist, desto höher ist die Auswahl. Die Bewegungen des selbstformenden Elementes sind nicht nur durch die Konstruktion sondern besonders durch das steuernde Computerprogramm bestimmt. Gerade weil der Bewegungsablauf aufgrund der Vielzahl der Aktuatoren und der Einlagerung dieser in ein elastisches Material nich vorausberechenbar ist, eignet sich ein solch selbstformendes Element zur Steuerung mit selbstlernenden Algorithmen. Ein solch gearteter elastischer Körper wird hierdurch muskelähnlich.

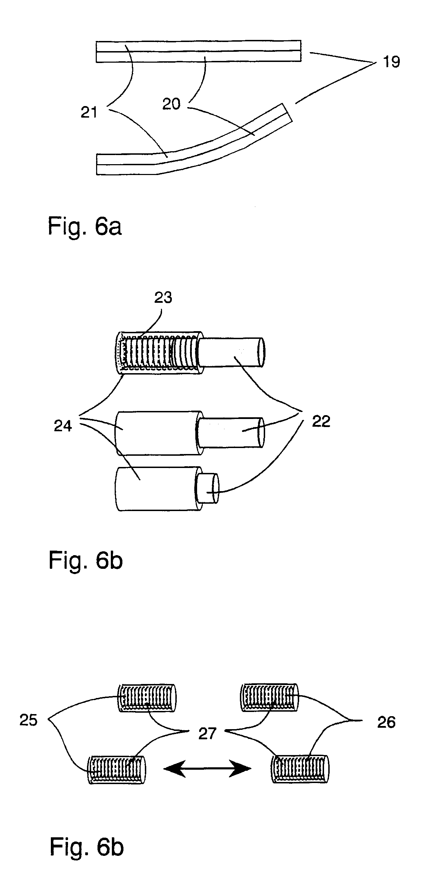

Die Aktuatoren können auch aus Thermobimetallen gefertigt sein. Als Beispiel ist in Fig. 6a ein solches Bimetall (19), bestehend aus einem Material (20) höherer Wärmeausdehnung als ein Material (21), gezeigt. In der oberen Teilfigur ist das Thermobimetall (19) ungeschaltet und daher ungebogen, in der unteren Teilfigur geschaltet und daher gebogen.

Die Aktuatoren können auch elektromagnetisch arbeiten. In Fig. 6b ist hierzu ein solcher Aktuator gezeigt. Dieser besteht aus einem magnetischen Kern (22), der durch das von einer Spule (23) erzeugte elektromagnetische Feld aus einer Hülle (24) gedrückt und wieder eingezogen werden kann. Im unteren Teilbild sind zwei Aktuatoren gezeigt. Das obere mit ausgestrecktem, das untere mit eingezogenem Kern.

Elektromagnetisch heißt auch, daß die einzelnen Aktuatoren selbst keine innere Bewegung ausführen, sondern sich einfach nur im geschalteten Zustand voneinander abstoßen oder einander anziehen. Dabei würde dann das zwischen ihnen liegende elastische Material gedehnt oder gestaucht. Fig. 6c zeigt hierzu zwei Aktuatoren (25) und (26), die sich im Schaltzustand der stromdurchflossenen Spulen (27), unteres Teilbild, abstoßen (Pfeil).

Die in dieser Schrift dargestellten Aktuatoren sind nur Beispiele. Aktuatoren, die sich drehen, winden, strecken, zusammenziehen, dehnen usw. sind denkbar, sowie solche aus dünnen Schichten und Folien. Die aktuatoren könnten auch piezoelektrisch oder elektrostriktiv arbeiten. Selbstformende Körper selbst könnten als Aktuatoren genutzt werden.

Zur Ermittlung der momentanen Stellung des selbstformenden Elementes können Drucksensoren oder Schalter in den elastischen Körper eingelassen sein. Die Drucksensoren messen dabei den inneren Druck des elastischen Körpers, der dann mit der Formveränderung interpretiert werden kann. Eine Vielzahl von elektrischen Schaltern, die nur den Zustand "offen" oder "geschlossen" kennen, ist für eine Rechnersteuerung einfacher, schneller und kostengünstiger.

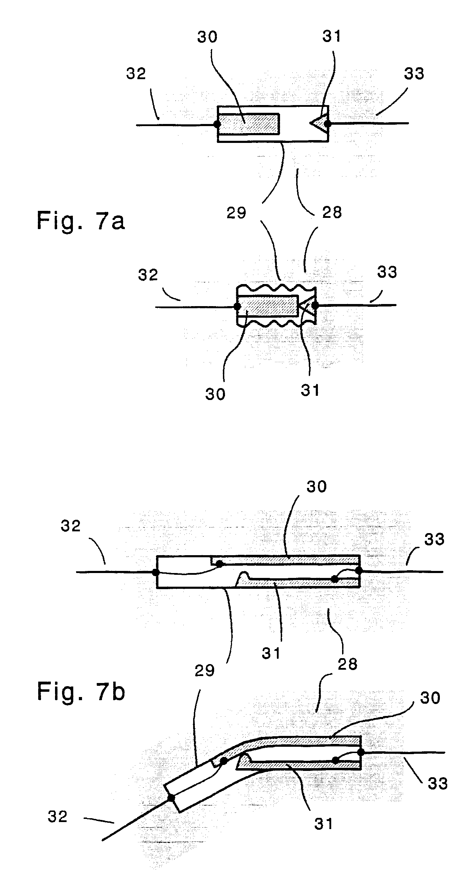

Fig. 7 zeigt hierzu mögliche Schalterkonfigurationen, die in das elastische Material (28) eingelassen sind. Die Schalter haben jeweils eine elastische Umhüllung (29) und zwei Schaltkontakte (30) und (31), an die die Signaldrähte (32) und (33) angeschlossen sind. Der Schalter im oberen Teilbild von Fig. 7a ist geöffnet, es fließt kein Strom. Im unteren Teilbild der Fig. 7a ist der elastische Körper (28) gestaucht, die Schaltkontakte berühren sich, so daß sich der Stromkreis schließt. In Fig. 7b wird der Kontakt geschlossen, wenn sich der elastische Körper (28) biegt - unteres Teilbild -. Die in Fig. 5 gezeigten Schalter (18) könnten wie die der Fig. 7b aufgebaut sein. Die geschlossenen Schalter (18') würden dann Aufschluß über die Biegung des Fingers (16) bzw. über die Formänderung des elastischen Körpers geben. Das Ergebnis ist genauer, je mehr Schalter vorliegen. Die Korellation der Schalterzustände mit der Biegung des Fingers (16) kann durch einen selbstlernenden Computeralgorithmus vorgenommen werden.

Durch die Vielzahl der Schalter bzw. Sensoren wird erreicht, daß die Stellung eines solchen Körpers durch selbstlernende assoziative Algorithmen ermittelt werden kann. Ähnlich wie beim z.B. Menschen, der bei schnellen dafür aber bekannten Bewegungen nicht mehr über die einzelnen Teilbewegungen nachdenken muß, bräuchten dann auch hier nur einige wenige exponierte Schalterstellungen abgefragt werden um assoziativ die Lage des Körpers zu erkennen. Die Vielzahl der Aktuatoren und Sensoren macht daher die Mechanik organischer.

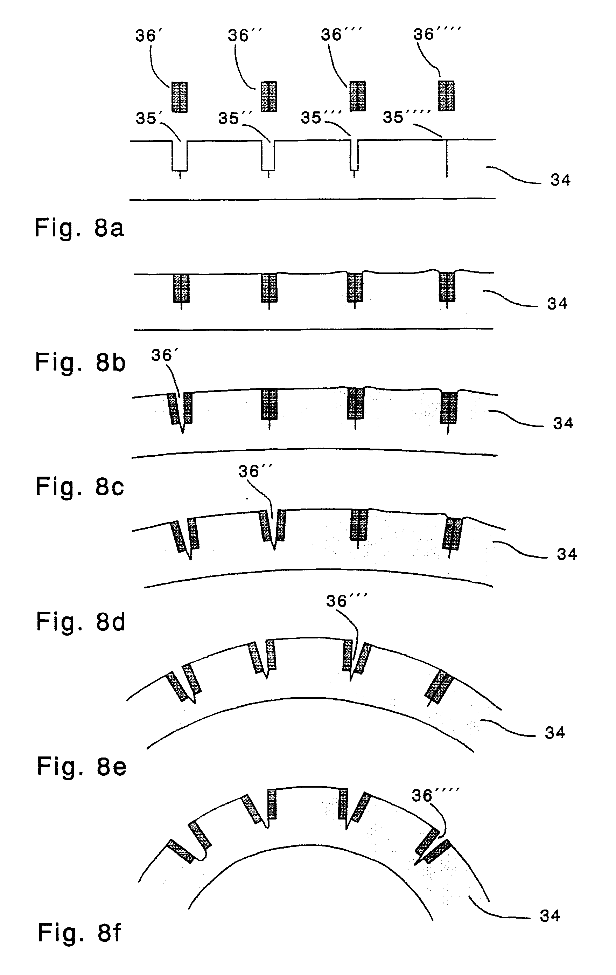

Eine andere Bauweise der Schalter ist im Querschnitt in Fig. 8 zu sehen. In Fig. 8a ist der elastische Körper (34) mit drei Materialaussparungen (35'), (35''), (35''') und dem bloßen Materialeinschnitt (35''') gezeigt. Die aus je zwei Schalterkontakten aufgebauten Schalter (36'), (36''), (36''') und (36'''') sind im Gegensatz zu den folgenden Figuren 8b - 8f in Fig. 8a zur Verdeutlichung noch nicht in den elastischen Körper (34) eingesetzt.

Im ungebogenen Grundzustand des elastischen Körpers (34) sind alle Kontakte (36) geschlossen, Fig. 8b. Wird der elastische Körper (34) gezeigt gebogen, so öffnen diese. Aufgrund der unterschiedlich großen Materialaussparungen und der in Fig. 8 gezeigten geometrischen Kurvenbiegung geschieht dies sukzessiv. In Fig. 8c (36'), in Fig. 8d (36''), in Fig. 8e (36''') und in Fig. 8f (36''''). Diese fortlaufenden Schalterstellungsänderungen können in einem Computerprogramm als Formänderung - Biegung - des elastischen Körpers (34) interpretiert werden.

Das Auslesen dieser Schaltervielzahl ist aufgrund der digitalen Schalterstruktur besonders einfach. Es kann ohne aufwendige Meßwertkorrekturen (Temperatur, Zeit, Hysterese) oder Analog-Digital-Konverter gearbeitet werden.

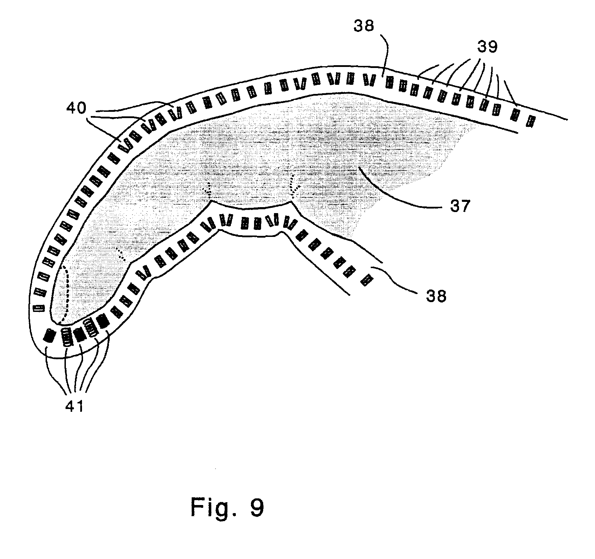

Das Prinzip der in Fig. 8 gezeigten Schalteranordnung kann für einen Datenhandschuh genutzt werden. Zur Verdeutlichung ist hierzu in Fig. 9 ein Fingerteil eines solchen Handschuhs im Querschnitt gezeigt. Der menschliche Finger (37) ist in den Fingerteil gesteckt, der aus einem elastischen Körper (38) besteht, in den eine Vielzahl von Schaltern (39) eingelagert sind. Je nach Biegung des menschlichen Fingers (37) ändert sich die Form des elastischen Körpers (38), so daß die Schalter geöffnet werden, wie z.B (40).

Die Bewegung der menschlichen Extremitäten kann so aufgenommen werden. Diese Schalterdaten können dann genutzt werden, um z.B. die Roboterhand der Fig. 5 zu steuern. Dabei könnte die Roboterhand ebenfalls einen exakt identisch aufgebauten Datenhandschuh tragen. Durch Vergleich aller Schalterstellungen beider Datenhandschuhe kann der Rechner die Roboterhand so bewegen, daß diese dieselben Bewegungen wie die der Menschenhand ausführt. Je höher die Anzahl der Schalter, desto genauer die Übereinstimmung.

Denkbar ist auch die Nutzung eines zweiten gleichgearteten elastischen Körpers zur Master-Slave-Steuerung.

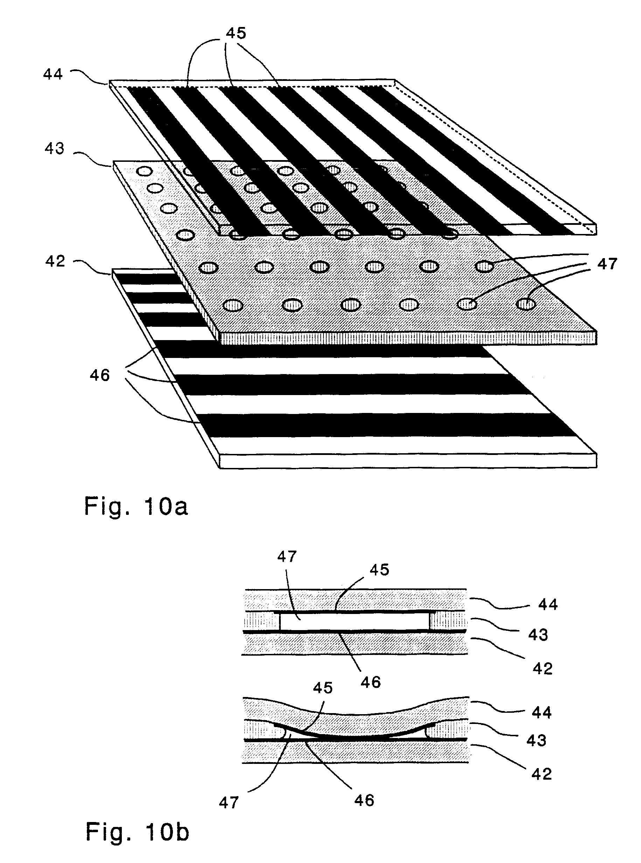

In Fig. 10 ist eine weitere Anwendung eines elastischen Körpers mit einer Vielzahl eingelagerter Schalter gezeigt. Der Körper besteht dabei aus drei elastischen, dünnen Platten (42), (43) und (44). Zum besseren Verständnis sind diese in Fig 10a in einer Explosionsdarstellung und Platte (44) in Durchsicht gezeigt. Sie liegen aber in der realen Struktur aufeinander und sind zu einem Körper verbunden. Auf die Platte (42) und unter die Platte (44) sind Metalldünnschichten aufgedampft, die so dünn sind, daß sie leichte Verformungen des elastischen Körpers bruchfrei mitmachen. Die Schichten sind zu Leiterbahnen (45) und (46) strukturiert, die wiederum rechtwinklig zueinander liegen. Die mittlere Schicht (43) ist genau dort mit durchgängigen Aussparungen (47) versehen, wo sich zwei Metalleiterbahnen der Platten (42) und (44) kreuzen.

Liegt dieser so geartete elastische Körper auf einem festen Untergrund - hier nicht gezeigt -, so ergibt sich an dieser Stelle ein Schalter. Dies wird an Fig. 10b deutlich, bei der der Querschnitt einer solchen Lochaussparung (47) gezeigt ist. Im oberen Teilbild der Fig. 10b liegen beide Metallbahnen um den Abstand der Platte (43) entfernt voneinander, es besteht kein elektrischer Kontakt. Durch Drücken auf die obere Platte (44) kontaktieren beide Leiterbahnen (45) und (46) miteinander und der Stromkreis ist geschlossen, unteres Teilbild der Fig. 10b. Entsprechende Entlüftungskanäle der Aussparungen (47) sind hier nicht gezeigt.

Durch Kenntnis der örtlichen Lagen der Lochaussparungen (47) kann ein so gearteter elastischer Körper zur ortsaufgelösten Druckmessung dienen. Solche Sensormatrizen können sich z.B. zum ortsaufgelösten Tasten an den Fingerspitzen der in Fig. 5 gezeigten Roboterhand befinden. Eine genauere Druckauflösung kann durch Übernanderlegung mehrerer solcher Sensormatrizen erreicht werden. Durch Schalten entsprechender Aktuatoren, die z.B. als Spiralfedern (41) ausgeführt sind, kann das so ermittelte Tastergebnis an die Fingerkuppe des menschlichen Fingers (37) im Datenhandschuh der Fig. 9 übertragen werden.

## Patentansprüche

1. Vorrichtung zum Greifen, die durch Aktuatoren verformt wird und aus elastichem Material gefertigt ist,
dadurch gekennzeichnet, daß
eine Vielzahl von dicht beieinander liegenden Aktuatoren (2, 10, 17) in das elastische Material eingebettet ist, die einzeln oder in Gruppen geschaltet werden können, so daß eine beliebige Formveränderung erzielt wird.

2. Vorrichtung nach Anspruch 1
dadurch gekennzeichnet, daß
die Vorrichtung die Form einer Hand aufweist.

3. Vorrichtung nach Anspruch 1 und 2
dadurch gekennzeichnet, daß
die Aktuatoren Formgedächtnismetalle (2, 10, 17) oder Thermobimetalle (19) sind oder elektromagnetisch, piezoelektrisch oder elektrostriktiv arbeiten.

4. Vorrichtung nach Anspruch 1 bis 3
dadurch gekennzeichnet, daß
die Formgedächtnismetalle spiralförmig (2, 17) gestaltet sind.

5. Vorrichtung zum Abtasten der Formänderung eines Körpers
dadurch gekennzeichnet, daß
diese aus einem elastischen Material (28, 34, 38) gefertigt ist, in das eine Vielzahl dicht beieinander liegender Schalter (18) oder Sensoren eingebettet ist und daß diese ihr Ausgangssignal ändern, wenn sich aufgrund der Formänderung des Körpers der innere Druck im umgebenden elastischen Material (28) ändert.

6. Vorrichtung nach Anspruch 5
dadurch gekennzeichnet, daß
der elastische Körper (28, 34, 38) zur Abtastung der Änderung der menschlichen Handform (37) und zur Eingabe dieser Änderung in ein Rechnersystem dient und die Form eines Handschuhs (38) aufweist, in den die menschliche Hand (37) eingelegt und in dem die menschliche Hand (37) bewegt wird.

7. Verfahren zur Verwendung einer Greifvorrichtung nach Anspruch 1 und einer Abtastvorrichtung nach Anspruch 5 und 6 nach dem Master-Slave-Verfahren
dadurch gekennzeichnet, daß
die Menschen- (37) und die Slave-Greifhand (16) identische Hand-Abtasthandschuhe (38) tragen und die Iststellung der Slave-Hand (16) durch direkten Vergleich beider ermittelt und die Sollstellung durch entsprechendes Nachsteuern erzielt wird.

8. Verfahren nach Anspruch 7
dadurch gekennzeichnet, daß
ein selbstlernender Rechenalgorithmus die Steuerung der Slave-Hand oder die Auswertung der Daten dem Vergleichs beider Handschuhe übernimmt.

## Claims

1. Prehensile device deformable by actuators and out of elastic material wherein a quaternary number of actuators (2, 10, 17) close to one another is embedded (integrated) into the elastic material, and said actuators are switched separatly or in groups in order to achiev any change of form.

2. Device according to claim 1 wherein
the device represent the form of a hand.

3. Device according to claim 1 and 2 wherein
the actuators are shape memory metals (2, 10, 17) or thermostatic bimetals and work electromagnetically, piezoelectrically or electrostrictially.

4. Device according to claim 1 to 3 wherein
the shape memory metals are realized spirally.

5. Device for scanning the change of form of a body wherein
said device is out of elastic material (28, 34, 38) in which is embedded a big number of switches (18) or sensors close to one another which changes their output-signal in case if the inner pression in the surrounded elastic material changes because of the change of form of the body.

6. Device according to claim 5 wherein
the elastic body (28, 34, 38) serves for scanning the change of form of the human hand (37) and for input of datas of the change in a computer and said elastic body has the form of a glove (38) in which the human hand (38) is put and moved.

7. Method for using a prehensile device according to claim 1 and a scanning device according to claimes 5 and 6 in accordance with the master-slave-method wherin
the human hand (37) and the slave hand (16) are completed by identical scanner gloves (38) and the real position of the slave hand (16) is determind by direct comparison and the nominal position is achieved by suppelementary adjustment.

8. Method according to claim 7 wherin
a self-learning algorithm controls the slave-hand and estimates the datas of comparison of the two gloves.

## Revendications

1. Dispositif de préhension déformable par des actionneurs et ètant realisé en materiau élastique caracterisé par le fait qu' un grand nombre d'actionneurs, disposés au dense voisinage l'un à l'autre (2, 10, 17), sont encastrés dans ce materiau elastique et peuvent être montés séparément ou en groupes, ce qui fait que tout changement de forme peut être obtenu.

2. Dispositif selon la revendication 1 caracterisé par le fait que le dispositiv a la forme d'une main.

3. Dispositif selon les revendications 1 et 2 caracterisé par le fait que les actionneurs sont réalisés en mètaux à mémoriser le changement de forme (shape memory metal) (2, 10, 17) ou en thermobimetaux (19) ou bien qui'ils fonctionnent d'une manière électromagnétique, pièzo-electrique ou électrostrictive.

4. Dispositif selon les revendications 1, 2 et 3 caracterisé par le fait que les metaux a mémoriser le changement du forme sont construits en spirales (2,17).

5. Dispositif pour le balayage du changement de forme d'un corps caracterisé par le fait qu'il est réalisé en materiau élastique (28, 34, 38) dans lequel un grand nombre de commutateurs (18) ou d'éléments sensibles, situés tès près l'un de l'autre, sont encastrés et que ces derniers changent leur signal de sortie en cas que la préssion interieure dans le materiau elastique environnant change à cause d'un changement de forme du corps.

6. Dispositif selon la revendication 5 caracterisé par le fait que le corps élastique (28, 34, 38) sert à balayer le changement de forme de la main humaine et à introduire des données de ce changement à un ordinateur et que ledit dispositif a la forme d'un gant (38), dans lequel la main humaine (37) est insérée est remuée.

7. Procédé pour l'utilisation d'un dispositif de préhension selon la revendication 1 et d'un dispositiv de balayage selon les revendications 5 et 6 d'apres le procédé bras esclave - bras maître (Master-Slave) caracterisé par le fait que la main humaine (37) et la main esclave (16) sont completées de gants de balayage identiques et que la position réelle de la main esclave (16) est déterminée par comparaison directe de positions de ces deux gants et la position nominale est obtenu par commande supplémentaire.

8. Procédé selon la revendication 7 caracterisé par le fait que la commande de la main esclave ou l'évaluation des données de la comparaison est effectué par un algorithme autoapprenant.
